# EUROPEAN PATENT APPLICATION

(11) **EP 2 481 350 A1**
(43) Date of publication of application: **01.08.2012**
(21) Application number: 12151740.3
(22) Date of filing: 19.01.2012
(51) Int. Cl.: A61B 5/055

(54) **Mr compatible stereoscopic viewing device for use in the bore of an mr magnet**

(30) Priority: 24.01.2011 US 201113012164
(71) Applicant: Imris Inc., Winnipeg, Manitoba R3T 1N5 (CA)
(72) Inventor: Schaerer, Shawn, Winnipeg, Manitoba R3T 1N5 (CA); Alexiuk, Mark, Winnipeg, Manitoba R3T 1N5 (CA); Scarth, Gord, Winnipeg, Manitoba R2N 2W4 (CA); Saunders, John K., Winnipeg, Manitoba R3T 1N5 (CA); Dahan, Meir, Winnipeg, Manitoba R3T 1N5 (CA)
(74) Representative: Jostarndt, Hans-Dieter

(57) **Abstract**

In an MR guided surgical system which is carried out in the bore of an MR magnet, there is provided a microscope system for viewing the required part of a patient which includes stereoscopic viewing components arranged for use in generating 2D and 3D images displayed to the surgeon. The optical assembly is adjustable to change the view and the visual images are overlaid by the MR images. The visual image can be adjusted in response to movement of the surgical tool and the MR image displayed and/or the image obtained can be modified in response to change in the visual image and/or movement of the tool. The components in the bore are made compatible with the MR environment.

## Description

This invention relates to an MR compatible stereoscopic viewing device for use in the bore of a magnet and to its cooperation with MR images and with a robot surgical system. The system may be used to generate combined fluorescence and MR images for improved surgery guidance in the resection of tumors.

### BACKGROUND OF THE INVENTION

In US Patent 7,155,316 (Sutherland) issued December 26 2006 is disclosed a system for Robotic microsurgery within the bore of an MR scanner. The disclosure of this patent is incorporated herein by reference or should be referred to for further details of the MR and robotic surgery system with which the present invention is concerned.

The above patent discloses a microsurgical robot system which is intended for use with an MR imaging system where the imaging magnet is retracted during the operating procedure since the system cannot function within the bore of the magnet. A disclosure of procedures within the bore is shown but this is limited to stereotactic procedures using one arm only and is limited by the fact that the microscope disclosed is not suitable for use within the bore thus precluding effective microsurgery.

That is the microsurgery is limited by the availability of a MR compatible stereoscopic microscope device with interactive high resolution to view the surgical site. In addition, line-of-sight issues exist in the following configuration: patient in the bore of the magnet; in-bore microscope surveying the surgical site; in-bore robotic arms between the surgical site and operating on the patient; other in-bore devices (camera, lights, HFD, etc). These line-of-sight issues may exist regardless of whether the surgeon is located in the OR or the control room. Therefore, the microscope needs to be compact and flexible for positioning. Sufficient lighting must also be available.

In addition, MR compatible microscopes are not available to allow fluorescence capabilities for viewing tumor boundaries.

The ability to combine MRI (for internal soft-tissue characterization) and microscopy (for high resolution dissection) and fluorescent microscopy (for enhanced tumor boundaries) along with an in-bore surgical robot is a powerful integration of technologies for neurosurgery.

Fluorescence imaging in neurosurgery has a long historical development, with various biomarkers and biochemical agents being used, and numerous technological approaches. This review focuses on contrast agents, summarizing endogenous fluorescence, exogenously stimulated fluorescence, and exogenous contrast agents, and then on tools used for imaging. It ends with a summary of key clinical trials that lead to consensus studies. The practical utility of protoporphyrin IX (PpIX) as stimulated by administration of δ-aminolevulinic acid has had substantial pilot clinical studies and basic science research completed. Recently, multi-center clinical trials using PpIX fluorescence to guide resection have shown efficacy for improved short-term survival. Exogenous agents are being developed and tested pre-clinically, and hopefully hold the potential for long-term survival benefit if they provide additional capabilities for resection of micro-invasive disease or certain tumor subtypes that do not produce PpIX or help delineate lowgrade tumors. The range of technologies used for measurement and imaging varies widely, with most clinical trials being carried out with either point probes or modified surgical microscopes. Currently, optimized probe approaches are showing efficacy in clinical trials, and fully commercialized imaging systems are emerging, which will clearly help to lead adoption into neurosurgical practice.

In a randomized phase III multi-center clinical trial using ALA-induced PpIX fluorescence as the tumor tissue contrast agent for neurosurgical guidance, tumor tissue was more completely resected and 6-month progression-free survival was extended.

Clinical use of microscopy has been largely limited to research trials, where mechanistic information is sought about the origins of uptake or localization.

### SUMMARY OF THE INVENTION

According to one aspect of the invention there is provided an apparatus for viewing a part of a patient comprising:
an optical assembly for receiving light from the part of the patient, the optical assembly including stereoscopic viewing components arranged for use in generating 2D and 3D images, the optical assembly being adjustable to change at least a field of view;
a display for viewing of images generated from the light received from the part;
and a control system for controlling the optical assembly and for generating the images;
a communication arrangement for communicating between the optical assembly and the processing system;
wherein the optical assembly includes a mount arranged to locate the assembly within a bore of an MRI magnet;
and wherein the optical assembly, control system and the communication arrangement are compatible with the MRI magnet so as to allow simultaneous communication and MR imaging.

Preferably the control system is arranged to change the viewing parameters of the optical assembly including one or more of zoom, depth of field, focus, pan, tilt, window levelling, color, balance, magnification.

Preferably an illumination source is integrated into the optical assembly to illuminate viewing of the part.

Preferably there is provided an imaging/encoding device or CCD for encoding light from the optical assembly from the part into digital information.

In one arrangement, the imaging/encoding device is located near the patient in the bore of a magnet at the optical assembly and the control system is located outside of the bore with communication therebetween using wires for communication the electrical signals carrying the image data.

In another arrangement, the imaging/encoding device is located remotely from the optical assembly and the communication arrangement comprises a fiber optic system.

In yet another arrangement, the imaging/encoding device is located remotely from the optical assembly and the communication arrangement comprises a light tube movable with the optical assembly.

Preferably the display is a remote display outside the bore so that the microsurgery is carried out by a surgeon at a remote operating location using robotic control of the effectors.

Preferably the display provides a visual real-time update of the surgical site and is combined with a real-time overlay of MR images for the real-time update of the stereoscopic display. That is the images from the MR system are registered with the visual images and overlaid to be viewed simultaneously by the surgeon.

In another arrangement, the display comprises a head mounted display for mounting on the surgeon.

In a yet further arrangement, the display is a traditional binocular setup or a stereoscopic 3D display. That is a conventional microscope type located at the bore for direct viewing of the site.

Preferably the optical assembly is sterilizable using conventional techniques.

Preferably the optical assembly, control system and the communication arrangement are made compatible with the MRI magnet by one or more of:
an RF filter on electrical communication cables to prevent stray RF from the electrical communication cables signals from effecting the imaging;
an RF filter on electrical communication cables to prevent the RF imaging signals from affecting the imaging/encoding device;
an RF enclosure around the optical assembly and the imaging/encoding device;
the optical assembly and imaging/encoding device being formed of materials which are compatible with the magnetic field;
cable traps on electrical communication cables to prevent heating thereof in the RF field of the imaging system;
a magnetic shield around or adjacent the components to prevent the magnetic field from affecting the components.

In one arrangement the optical assembly is mounted by the mount to the bore.

In another arrangement the optical assembly is mounted by the mount on an arm extending into the bore.

Preferably the apparatus is used with a surgical robot system including at least one robotic arm with at least one end effector for operating one or more surgical tools.

In this arrangement the optical assembly can be mounted on the robotic arm so as to be moveable therewith, that is the optical assembly is mounted on the robotic arm so as to movable with the tool and so as to have a field of view including a tip of the tool.

In another arrangement the optical assembly is mounted on the tool so as to movable with the tool and so as to have a field of view including a tip of the tool or the optical assembly can be mounted on the tool at a tip of the tool.

Preferably the optical assembly can be mounted on a support arm separate from the robotic arm or arms so as to be moveable with the support arm where the support arm is controlled to avoid interference with the robot arms which move to effect the surgical procedures.

Preferably the control system is arranged to operate the optical assembly to change one or more of the viewing parameters thereof in response to movement of the robot arm.

Alternatively the control system is arranged to operate the optical assembly to change one or more of the viewing parameters thereof in response to movement of the tool relative to the optical assembly.

For example the control system is arranged to operate the optical assembly to change the focal position thereof in response to movement of the tool relative to the optical assembly to focus in the area of the tool tip.

For example the control system is arranged to operate the optical assembly to change the focal depth thereof in response to movement of the tool relative to the optical assembly.

Preferably the robot arm or arms are controlled to be moved in response to input from a user and the movement of the arm or arms is scaled in relation to the image displayed.

Preferably the robot arm or arms are controlled to be moved in response to input from a user and the movement of the arm or arms is limited by no-touch zones indicated on the image.

Preferably the MRI system is arranged to generate MR images and the control system is arranged to cooperate with a control system of the MRI in order to overlay the MR images on the visual images on the display.

In this arrangement, preferably the control system of the MRI is arranged to operate in response to changes in the visual image displayed.

In this arrangement, preferably the control system of the MRI is arranged to change the MR images acquired for overlay in response to changes in the visual image displayed.

In an arrangement where the apparatus is used with a surgical robot system including at least one robot arm with at least one end effector for operating one or more surgical tools, preferably the control system of the MRI is arranged to operate in response to movement of the tool and/or to changes in the visual image displayed.

Preferably the control system of the MRI is arranged to change one or more of:
the scan parameters of the MR images including one or more of resolution, slice thickness and dimension;
the scan type(T1,T2, etc);
the part of the patient / anatomy is being scanned based on either the visual image changing or the tool moving.

For example the control system of the MRI is arranged to trigger a scan.

For example the control system of the MRI is arranged to change the MR imaging from detecting a position of the tool tip to providing an anatomical image.

Preferably the control system is integrated with an IGS system which can overlay or augment the surgeon's view with image guidance data.

Preferably the optical assembly is arranged to detect visual images, IR images and/or florescence.

Preferably the optical assembly is arranged for receiving light from the part of the patient including visible light and florescent light emitted from fluorescing cells within the part, wherein the MRI system is arranged to generate MR images and wherein the control system is arranged to cooperate with a control system of the MRI in order to overlay the MR images on the visual images including the florescent light on the display.

Preferably the combination of quantitative fluorescence imaging with MR imaging is used to determine the amount of residual tumor present during a neurosurgical procedure, to guide the surgeon or the surgical robot in the resection of this residual tumor and guarantee that normal brain is left intact.

Preferably the patient receives a drug which enters brain tumor cells exclusively and fluoresce once resident therein.

Preferably the fluorescence is analyzed quantitatively, such that, since the fluorescent drug resides exclusively in the tumor cells, the quantitative measurement of the drug concentration is a measure of the concentration of tumor cells.

Preferably the MR imaging is used to provides a more complete picture of the location of tumor cells present.

Preferably the image representing the residual tumor mass is segmented and the data transferred to the robot which is used to resect the tumor to the level assigned by the quantitative analysis of the fluorescent images.

Preferably the MR images which are co-registered with the fluorescent images are involved in the segmentation to provide tumor cell zones and also keep out zones related to eloquent and sensitive brain structures.

Preferably the imaging rate for the fluorescence imaging is of the order of 30 frames per second so it allows the resection to be monitored as it occurs.

Preferably the robot is programmed to stop as each MRI image is recorded.

Preferably the MR imaging includes diffusion tensor imaging which shows on the image all the fiber tracks in the brain and of particularly importance, those around the tumor.

Preferably the fluorescent chemicals which attach to the tumor cells also contain MRI markers so that they appear on both the MR images and the fluorescence images.

Preferably the registration of the images is achieved by placing markers on a tool of the robot or surgical instrument.

According to a second aspect of the invention there is provided an apparatus for viewing a part of a patient comprising:
an optical assembly for receiving light from the part of the patient including visible light and florescent light emitted from fluorescing cells within the part;
a control system for generating from the light received a visual image of the part and including thereon the florescent light;
a display for viewing of the visual images generated from the light received from the part, the display including the florescent light;
a mount arranged to locate the optical assembly within a bore of an MRI magnet;
wherein the optical assembly, control system and the communication arrangement are compatible with the MRI magnet so as to allow simultaneous communication and MR imaging; and
wherein the MRI system is arranged to generate MR images and wherein the control system is arranged to cooperate with a control system of the MRI in order to overlay the MR images on the visual images including the florescent light on the display.

The invention includes an MR compatible microscope that provides a surgeon with the ability to view a surgical site in 2D or 3D either locally or remotely at a remote viewing station, along with a novel mounting mechanism that is optimized for use in the bore of an imaging magnet (e.g., in combination with a surgical robot in the bore).

The device can have controls which give the surgeon the ability to change the viewing parameters of the microscope including zooming, depth of field, focus, pan, tilt, window levelling, color, balance, etc. Surgical lighting can be integrated into the microscope to allow viewing of the surgical site. The device can be integrated with IGS system which can overlay or augment the surgeon's view with image guidance data.

The microscope system contains an imaging/encoding device, a processing device, and one or more display devices. The imaging/encoding device is located near the patient in the bore of a magnet, whereas the processing device is located outside of the RF shield. The display devices can be located within or outside of the RF shield. For example, to support remote viewing as may be needed with a surgical robot, the display device can be located in a control room.

While the MR is imaging (e.g., real-time update of MR images for use in surgery), the MR compatible microscope can produce the stereoscopic display for the corresponding visual real-time update of the surgical site. These can also be combined for real-time overlay of the MR images for the real-time update of the stereoscopic display.

The stereoscopic signals are displayed on screens for both 2D and 3D displays, and can optionally be sent to a head mounted display. The output of the microscope can be a traditional binocular setup or be part of a stereoscopic 3D display either with or without 3D glasses. Video can also recorded and archived.

The microscope is minimally sized to allow it to be located in the bore of an imaging magnet, and can contain the following characteristics:
Different magnification levels that are adjustable from a remote location (outside of bore)
   F ocusing capabilities from a remote location (outside of bore) Integrated surgical lighting.
   F luorescent viewing support.

Since the components of the microscope can be near the surgical field, they are sterilizable.

For MR compatibility the system can:
Include integrated optics and digitizing in a device located in the bore
Use optics including lenses with fiber optics in the bore to route the optical signals out of the bore, with all electronics mounted outside of the bore and RF shielded
Use MR compatible circuits and imaging chips mounted at the tip of the device (where the optics are located, that is, chip in the tip).

Use images which are digitally encoded.

The 3D image from the MR compatible microscope can be integrated into any application where a tradition microscope view is integrated.

This device can work standalone or can be part of a surgical robotic system.

The MR compatible microscope can be mounted on apparatus that allow the microscope to be positioned over a patient in the bore of the magnet, such as free-standing on the OR floor and extend the microscopy into the bore, mounted to the magnet bore or other equipment in the bore.

The microscope can also be mounted onto a surgical robot, for example, mounted onto or near the end-effectors of a robot arm. If two robot arms operate in the bore, then each arm could have a separate microscope. The microscope can also be combined and integrated directly with the tools or instruments which are attached to the robot end effector(s). This can also be combined with a microscope display independent of a robot.

The system can provide the surgeon with the ability to view in either 2D or 3D the surgical site in the bore of an MRI either remotely or locally, this enables microsurgery to take place in the bore of an MRI, for example, in combination with a surgical robot

Attaching the microscope to each end effector and/or integrated tool of a robot working in the bore provides the surgeon with view of the surgical site which is always inline with the surgical tool. For example, the microscope can be mounted under, on top of, or beside the tools/end effector to maximizes the viewing capabilities of the device while minimizing the interference with performing surgery.

The small size allows multiple microscopes to be used for the same surgery in the bore.

The arrangement described above can also be used for the Detection and Removal of residual Brain Tumors during Neurosurgery.

Intra cranial malignant brain tumors are the most common and aggressive primary tumors in the central nervous system and carry one of the worst prognosis of all types of cancers. Radical surgical resection is the major treatment for these tumors and this is often supplemented with radiation treatment and/or chemotherapy. The goal of the surgical procedure is to remove all of the tumor tissue or at least as much as possible without causing any neurological deficit to the patient. Most brain tissue is eloquent and so the surgeon cannot remove any normal brain tissue since this could result in neurological dysfunction. The advent of intra operative MRI has increased the surgeon's ability to increase the amount of resection without removing normal brain tumor. Intra operative MRI also eliminates the problem of brain shift which can make navigation equipment inaccurate and therefore of limited utility in defining tumor boundaries.

Another method for detecting residual tumor cells during surgical resection which has been developed is to use fluorescence imaging of chemicals which attach only to the tumor cells and which fluoresce under optical irradiation. This optical image when taken during the surgical procedure provides the location of tumor cells within the surgical cavity. This can be achieved in real time but is only visible at the surface of the residual tumor tissue. The optical image does not provide information as to what is just below the surface which may be highly sensitive brain tissue. MRI imaging is not as sensitive as fluorescent imaging and therefore a larger number of cancer cells need to be present for detection by MRI.

The invention is to combine quantitative fluorescence imaging with MR imaging determine the amount of residual tumor present during a neurosurgical procedure, to guide the surgeon or the surgical robot in the resection of this residual tumor and verify that normal brain is left intact. The surgical procedure can be performed in the operating room equipped with a moveable MRI magnet capable of moving over the magnet for imaging at the appropriate time or in the bore of the magnet when in an MRI equipped operating theatre.

The patient receives a drug which enters brain tumor cells exclusively and fluoresce once they are resident therein. The fluorescence is monitored at the appropriate frequency by an operating microscope or a camera. The fluorescence is analyzed quantitatively. Since the fluorescent drug resides exclusively in the tumor cells the quantitative measurement of the drug concentration is a measure of the concentration of tumor cells. The drug does not enter all the tumor cells but the concentration is representative.

The MR imaging provides a much more complete picture of the amount of tumor cells present. Co-registration of the MR images to the fluorescent images is used to calibrate the fluorescent images. It is well known that tumors are very heterogeneous with the heterogeneity being both in terms of tumor cells and even more importantly in the grade. The grade is a measure of the malignancy of a tumor and ranges from 1 to 4 with 4 being the most malignant. Often the ability of the fluorescent drugs to enter a cell depends on its grade and some drugs may just enter and hence mark only a specific grade of tumour, for example only grade 4 tumors. The MRI image is normally relatively independent of grade and therefore provides a more complete picture of the cancer cell population.

The image representing the residual tumor mass can be segmented and the data transferred to the robot and the robot resect the tumor to the level assigned by the quantitative analysis of the fluorescent images. The resection of the tumor mass is monitored by the rapid fluorescent imaging. The MR images which are co-registered with the fluorescent images are involved in the segmentation to provide tumor cell zones and also keep out zones related to eloquent and sensitive brain structures. These zones are registered for both MR and Fluorescent guided resection. The imaging rate for the fluorescence imaging is of the order of 30 frames per second so it monitors the resection as it occurs. The resection can be performed out of the magnet by the surgeon or in the magnet using the robot. The robot can resect the tumor automatically using the segmented images updated by the rapid fluorescent imaging. MRI in the brain can not reach these rates and each image of reasonable resolution takes about 5 seconds so that the robot should be programmed to stop as each MRI image is recorded. The quantization of the fluorescence images guides the robot. As stated above the robot can resect automatically or can be guided by the surgeon as to which tissue should be removed. The MRI shows the surgeon the proximity of sensitive brain tissue to the tool held by the robot and permits the robot to resect much closer to the sensitive brain tissue. There are many types of tissue but a good example is the optic nerve. Cancer tissue can essentially wrap itself around the nerve. The surgeon wishes to resect as much tissue as possible but not have any deleterious effect on the nerve function. The combination of real time fluorescence and rapid MRI permits maximum resection of the tumor tissue without impacting nerve function. MR Imaging in the operating room using diffusion tensor imaging shows all the fiber tracks in the brain and of particularly importance, those around the tumor. Again, the combined imaging techniques allows the robot to resect any tumor tissue very close to these tracks without impacting the functionality of the tracks. The resection of tumors close to sensitive areas can be controlled by the surgeon or can be controlled by programming keep out zones as described above.

The fluorescent chemicals which attach to the tumor cells can also contain MRI markers so that they appear on both the MRI images and the fluorescence images. The two sets of images need to be registered to one another and this can be achieved by placing appropriate markers on a tool of the robot or a surgical instrument.

In summary the combination of the two imaging technologies takes advantage of the high sensitivity, the rapid imaging and ease of acquisition capabilities of fluorescence with the three dimensional and independence of tumor grade imaging of MRI. MRI also provides a significant level of safety for normal brain tissue during the resection.

The advantage of the invention is that it results in increased resection percentage and decreased neurological deficit. It is the combination of intra operative MRI, intra operative fluorescence and the robot which makes this unique and innovative. The aim is always to improve patient outcome and having the imaging technologies essentially simultaneous available when the surgeon (robot) performs the surgery leads to the best result. The fluorescence imaging brings the dimensions almost to the cellular level and the robot is much more accurate than any human surgeon.

This invention bring together real time imaging at a cellular level of tumor cells with almost real time imaging of the environment of these tumor cells in the human brain at the time when the surgeon is actually performing the surgical procedure. This means that the surgeon knows precisely where the tumor cells that are being resected are and their location relative to the all other parts of the brain. The addition of MRI to fluorescence imaging permits all the tumor mass to be detected even when the tumor is heterogeneous. The combination of fluorescent imaging with MRI imaging allows imaging to monitor tumor resection both in the bore and out of the bore. The use of the robot to perform the procedure allows the tumor to be resected in the bore of the magnet with both MRI and fluorescence imaging. The combination of both imaging modalities when processed enables the system to define both the target tissue and the no go zones to the robot or the surgeon.

### BRIEF DESCRIPTION OF THE DRAWINGS

One embodiment of the invention will now be described in conjunction with the accompanying drawings in which:
Figure 1 is a schematic side elevational view of a microsurgical robot system operating with the bore of an MR magnet with a viewing device for real time viewing of the tools and operation site which can be overlaid with real time MTR images from the imaging system.
Figure 2 is a schematic side elevational view similar to that of Figure 1 showing a different mounting for the viewing system.
Figure 3 is a schematic view showing the viewing system.
Figure 4 is a schematic side elevational view of one robot arm of the system of Figure 1 with the viewing device mounted on the tip of the tool.
Figure 5 is a schematic side elevational view of the end effector of one robot arm of the system of Figure 1 with the viewing device mounted on the end effector adjacent the tool.
Figure 6 is a schematic illustration of an MR image and visual image controlled micro-surgery system.

In the drawings like characters of reference indicate corresponding parts in the different figures.

### DETAILED DESCRIPTION

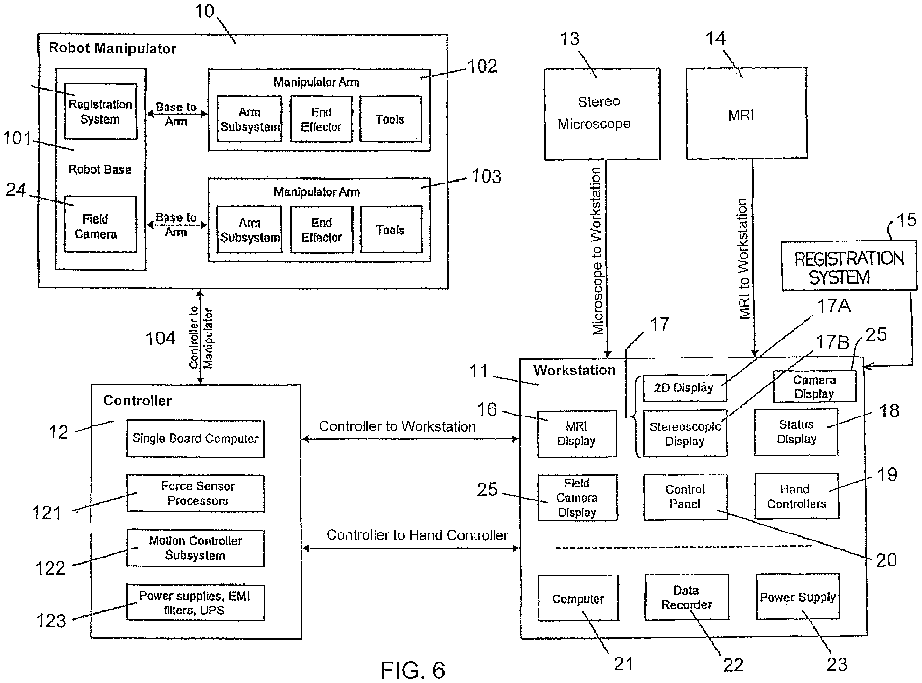

An overview of the system is shown in Figure 6 which comprises a robot manipulator 10, a work station 11 and a controller 12 which communicates between the robot manipulator and the work station. As an input to the work station is also provided a stereo microscope 13, an MRI imaging system 14 and a registration system 15.

The work station includes a number of displays including at first display 16 for the MRI image, a second display 17 for the microscope image and a third display 18 for the system status. Further the work station includes two hand controllers schematically indicated at 19 and an input interface 20 allowing the surgeon to control the systems from the work station while reviewing the displays. The work station further includes a computer or processor 21, a data recording system 22 and a power supply 23.

The display 17 includes a stereoscopic display 17A which provides a simulated microscope for viewing the images generated by the stereo-microscope system 13. Further the display 17 includes a monitor 17B which displays a two dimensional screen image from the microscope system 13.

The robot manipulator 10 includes a field camera 24 which provides an image on a monitor 25 at the work station.

The magnetic resonance imaging system 14 is of a conventional construction and systems are available from a number of manufacturers. The systems are of course highly complicated and include their own control systems so that the present workstation requires only the display of the image on the monitor 16 where that image is correlated to the position of the tool using known registration systems.

The hand controllers 19 are also of a commercially available construction available from a number of different sources and comprise 6 degrees of freedom movable arms which can be carefully manipulated by the surgeon including end shafts 19A which can be rotated by the surgeon to simulate the rotation of the tool as described hereinafter. An actuator switch on the tool allows the surgeon to operate the actuation of the tool on the robot as described hereinafter.

The robot manipulator comprises a cabinet 101 and two arms 102 and 103 which are mounted on the cabinet together with the field camera 24 which is also located on the cabinet. The field camera is mounted at the back of the cabinet viewing past the arms of the front of the cabinet toward the patient and the site of operation to give a general overview field of the situation for viewing on the display 25.

The control system 12 for communication between the work station and the robot manipulator and for controlling the operation of each of those components includes a force sensor sub system 121 and a motion control sub system 122 together with power supplies and further components as indicated schematically at 123. The force sensor sub system controls the feed back forces as detected at the end effector of the robot arm to the hand control systems 19. The motion control subsystem 122 converts the motion control sensors from the handcontrol system 19 into individual operating instructions to the various components of the arms. The motion control sub system also provides an output which is communicated to the work station for display on the MRI imaging monitor 16 of the location of the tip of the tool relative to the image displayed on the screen 16, as generated by the registration system 15.

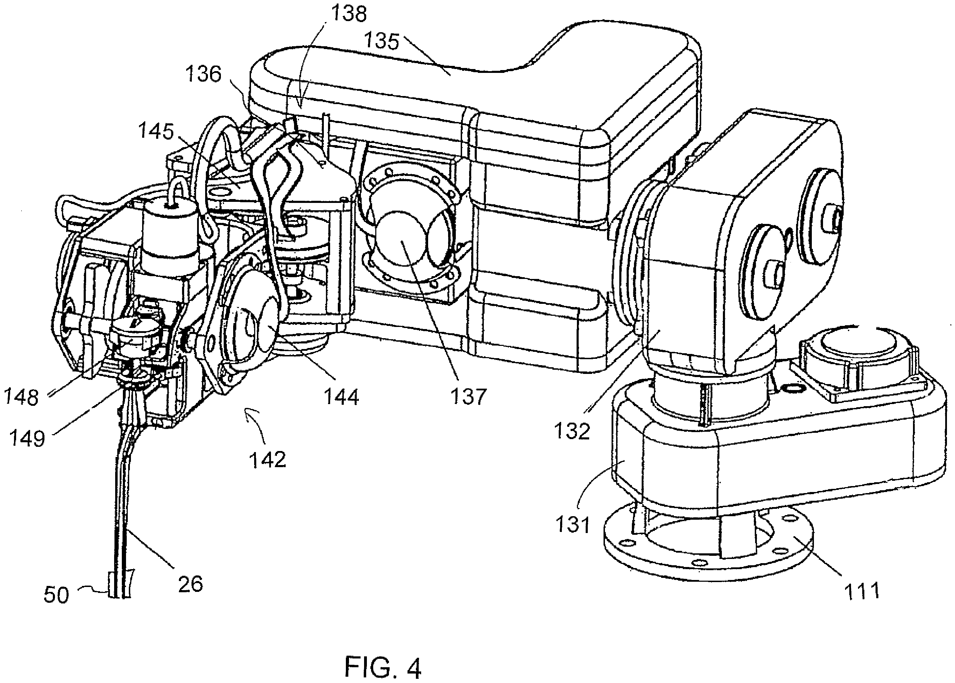

The structure of the arms is shown schematically in Figure 4, where the arms are mounted with their base 111 for attachment to the cabinet support. Each of the arms 102 and 103 includes a number of joints which allow operation of a tool schematically indicated at 26. Thus each arm includes a first joint defining a shoulder yaw pivot 131 defining a vertical axis of rotation. On the vertical axis is mounted a second joint 132 forming a shoulder roll joint which provides rotation around a horizontal axis. The shoulder yaw axis extends through the joint 132. A rigid link 135 extends from the joint 132 to an elbow joint 136 which is cantilevered from the shoulder roll joint 132. The elbow joint includes an elbow yaw joint 137 and an elbow roll joint 138. The yaw joint 137 is connected to the outer end of the link 135 and provides rotation about a vertical axis. The roll joint 138 is located on the axis and provides a horizontal axis. A link 141 lies on the horizontal axis and extends outwardly from the joint 138 to a wrist joint generally indicated at 142. The wrist joint 142 includes a wrist yaw joint and wrist roll joint. The wrist yaw joint provides a vertical axis about which a link can pivot which carries the roll joint. The roll joint provides a horizontal axis which allows the tool 26 to rotate around that horizontal axis. The tool 26 includes a roll joint 148 which provides rotation of the tool 26 around its longitudinal axis. The tool further includes a tool actuator 149 which can move longitudinally along the tool to provide actuation of the tool using various known tool designs.

Thus the forces required to provide rotation around the various axes are minimized and the forces required to maintain the position when stationary against gravity are minimized. This minimization of the forces on the system allows the use of MRI compatible motors to drive rotation of one joint component relative to the other around the respective axes.

The arrangement described above allows the use of piezoelectric motors to drive the joints. Such piezoelectric motors are commercially available and utilize the reciprocation effect generated by a piezoelectric crystal to rotate by a ratchet effect a drive disc which is connected by gear coupling to the components of the joint to effect the necessary relative rotation.

The robot therefore can be used in the two arm arrangement for microsurgery in an unrestricted area outside of the closed bore magnet or for microsurgery within an open bore of a magnet where the arrangement of the magnet can be suitable to provide the field of operation necessary for the two arms to operate. The two arms therefore can be used with separate tools to effect surgical procedures as described above. In some cases a single arm can be used to effect stereotactic procedures including the insertion of a probe or cannula into a required location within the brain of the patient using the real time magnetic resonance images to direct the location and direction of the tool.

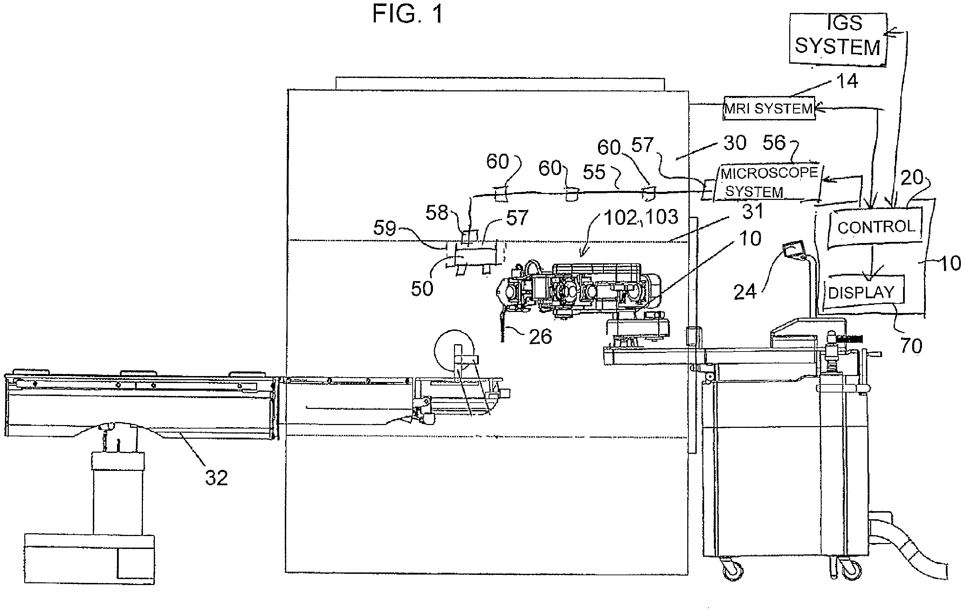

In Figure 1, the system is shown schematically in operation within the bore of a magnet 30 of the MRI system 14. The bore 31 is relatively small allowing a commercially available patient table 32 to carry the required portion of the patient into the bore to the required location within the bore. The field camera is used within the bore for observing the operation of the robot 10 and particularly the tool 26.

The stereo microscope system of the present invention as shown in mounted on a suitable support adjacent the patient for viewing the necessary site. The stereo microscope includes two separate imaging systems one for each channel which are transmitted through suitable connection to the display 17 at the work station. Thus the surgeon can view through the microscope display 17A the three dimensional image in the form of a conventional microscope and can in addition see a two dimensional image displayed on the monitor 17B.

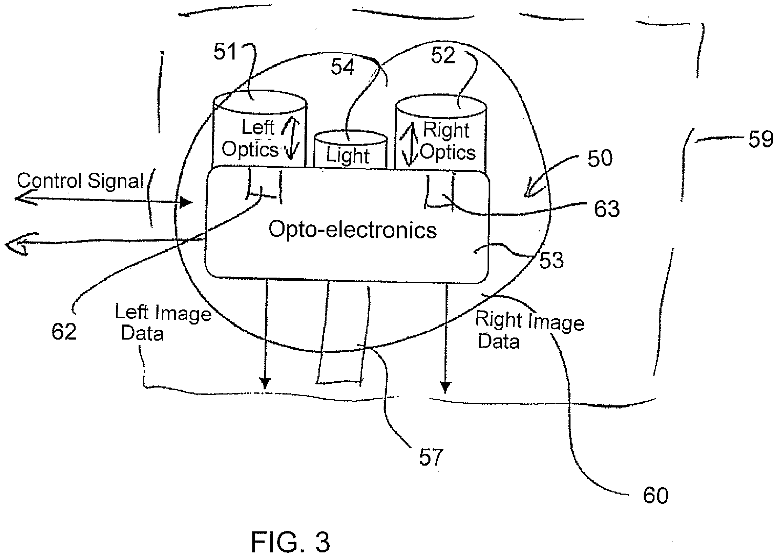

The stereo microscope system shown in Figure 3 comprises an optical assembly 50 for receiving light from the part of the patient, the optical assembly including stereoscopic viewing components 51 and 52 arranged for use in generating 2D and 3D images as described above.

The optical assembly is adjustable using the opto-electronics 53 to adjust one or more of the field of view, zoom, depth of field, focus, pan, tilt, window levelling, color, balance, magnification in response to control signals from a remote controller 56.

An illumination source 54 is integrated into the optical assembly to illuminate viewing of the part.

The light images from the left and right optics are converted using a CCD or similar system at the electronics system 53 into digital electrical signals which are transmitted to the microscope control system 56 for display on the display 70 of the work station 10 for viewing of images generated from the light received from the part. The control system 56 acts to control the optical assembly and for generating the images. A communication arrangement in the form of a cable 55 is provided for communicating between the optical assembly 50 and the processing system 56 outside the bore.

The optical assembly 50 includes a mount 57 arranged to locate the assembly within a bore of an MRI magnet. In Figure 1, the mount is arranged to attach the assembly to a fixed position within the bore at a location where it does not interfere with the arms 102, 103. The optical assembly 50 is sterilizable using conventional techniques.

The optical assembly, control system and the communication arrangement are arranged to be compatible with the MRI magnet so as to allow simultaneous communication and MR imaging. This is achieved by the following:
an RF filter 57 on the electrical communication cable 55 to prevent stray RF from the electrical communication cables signals from effecting the imaging;
an RF filter 58 on the electrical communication cable 55 to prevent the RF imaging signals from affecting the imaging/encoding device;
an RF enclosure 59 around the optical assembly and the imaging/encoding device;
the optical assembly 50 and imaging/encoding device 53 being formed of materials which are compatible with the magnetic field;
cable traps 60 on the electrical communication cable 55 to prevent heating thereof in the RF field of the imaging system;
a magnetic shield 61 around or adjacent the components to prevent the magnetic field from affecting the components.

In Figure 1, the imaging/encoding device or CCD 53 is located near the patient in the bore of a magnet at the optical assembly and the control system 56 is located outside of the bore with communication therebetween using wires for communication the electrical signals carrying the image data.

The display 70 is a remote display outside the bore so that the microsurgery is carried out by a surgeon at the remote operating location 10 using robotic control of the effectors. The display 70 provides a visual real-time update of the surgical site and is combined with a real-time overlay of MR images for the real-time update of the stereoscopic display. That is the images from the MR system are registered with the visual images and overlaid to be viewed simultaneously by the surgeon. The visual images and also the MT images are also controlled as explained hereinafter so that the views are compatible with each other and with the operation of the tools 26.

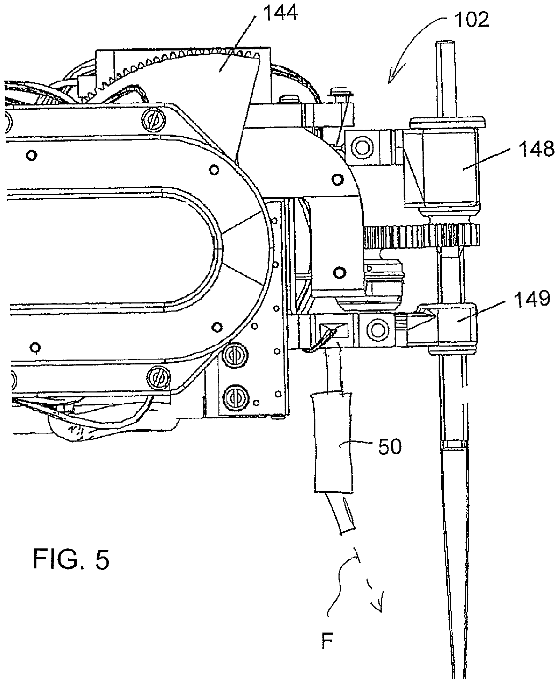

The apparatus is used with the surgical robot system described above. In Figure 5 the optical assembly is mounted on the robotic arm 102 or two separate systems are mounted on respective ones of the arms 102 and 103 so as to be moveable therewith. That is the optical assembly is mounted on the robotic arm so as to movable with the tool and so as to have a field of view F including a tip of the tool.

In Figure 4 the optical assembly 50 is mounted on the tool at a tip of the tool. In this case the assembly can be made much smaller to be movable with the tip possibly endoscopically.

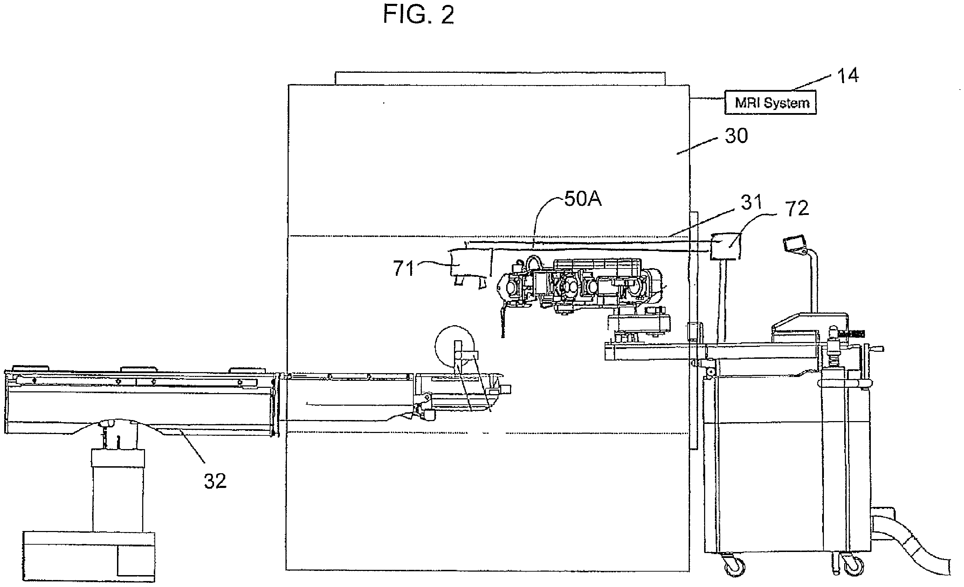

In Figure 2, the optical assembly 50 is mounted on a support arm 50A separate from the robotic arm or arms so as to be moveable with the support arm 50A where the support arm 50A is controlled in its movement to avoid interference with the robot arms 102, 103 which move to effect the surgical procedures.

As explained previously, the control system 53 includes motors 62, 63 arranged to operate the optical assembly to change one or more of the viewing parameters thereof in response to movement of the robot arm or in response to movement of the tool relative to the optical assembly. For example the control system is arranged to operate the optical assembly using the motors 62, 63 to change the focal position thereof in response to movement of the tool relative to the optical assembly to focus in the area of the tool tip. For example the control system is arranged to operate the optical assembly using the motors 62, 63 to change the focal depth thereof in response to movement of the tool relative to the optical assembly.

In addition, the robot arm or arms 102 and 103 are controlled to be moved in response to input from a the surgeon with the movement of the arm or arms being scaled in relation to the image displayed. This is obtained by providing information from the display 70 and the system 56 to the motion controller sub system 122 so that the amount of movement of the tool in response to an input from the hand controllers 19 changes, that is increases or decreases depending on the size of the image displayed ad the display 70. This assists the surgeon in controlling the movement since the amount of movement he achieves by moving the hand controllers by a predetermined distance matches the amount of movement viewed regardless of the scale of the displayed image.

In addition, the surgeon can set up in the image a no-touch zone which are intended to never be entered by the tool in view of potential damage to the patient. The controller 122 is arranged to operate robot arm or arms so that they are controlled to be moved in response to input from a user while preventing their entering the no-touch zones indicated on the image. This requires coordination of the processing programs of the control system 122 and the imaging system 56.

In addition the MR control system 14 is arranged to operate the MR imaging in response to changes in the visual image displayed as controlled by system 56 and in response to movement of the tool as controlled by system 122.

For example, the MR control system 14 is arranged to control, in response to changes in image and/or movement of the tool, the scan parameters of the MR images including one or more of resolution, slice thickness and dimension; the scan type(T1,T2, etc); the part of the patient / anatomy. In addition the control system 14 can be used to trigger a scan in response to a change in image displayed and/or the movement of the tool.

In addition the control system 14 of the MRI is arranged to change the MR imaging from detecting a position of the tool tip to providing an anatomical image.

In addition the control system is integrated with the IGS system which can overlay or augment the surgeon's view with image guidance data.

Preferably the optical assembly is arranged to detect visual images, IR images and/or florescence. These can be converted to visual images displayed on the display

Where the imaging/encoding device is located remotely from the optical assembly as shown in Figure 2, the communication arrangement between the optical portion 71 and a remote CCD 72 is provided by a fiber optic system or a light tube movable with the optical assembly. The use of a light tube or a fiber optic avoids the possibility of interference between the RF signals of the MR system and any RF field generated by the signals in the cable of Figure 1.

In another arrangement, the display can be provided as a head mounted display for mounting on the surgeon.

The arrangement described above can also be used for the Detection and Removal of residual Brain Tumors during Neurosurgery. The arrangment combines quantitative fluorescence imaging with MR imaging determine the amount of residual tumor present during a neurosurgical procedure, to guide the surgeon or the surgical robot in the resection of this residual tumor and verify that normal brain is left intact. The surgical procedure can be performed in the operating room equipped with a moveable MRI magnet capable of moving over the magnet for imaging at the appropriate time or in the bore of the magnet when in an MRI equipped operating theatre.

Thus in this arrangement, as explained above, the optical assembly, control system 56 and the communication arrangement 55 are compatible with the MRI magnet so as to allow simultaneous communication and MR imaging and the MRI system is arranged to generate MR images and the control system 56 is arranged to cooperate with a control system 20 of the MRI in order to overlay at the display 70 the MR images on the visual images including the florescent light on the display.

The apparatus is used with a surgical robot system 10 including at least one robotic arm 102, 103 with at least one end effector 149 for operating one or more surgical tools 26. The control system 56 of the imaging system is arragned to generate quantitative information relating to the amount of light emitted in the fluorescence. The combination of quantitative fluorescence imaging with MR imaging is used to determine the amount of residual tumor present during a neurosurgical procedure, and to display this on the display 70 to guide the surgeon or the surgical robot in the resection of this residual tumor and verify that normal brain is left intact.

In the operation, the patient receives a drug which enters brain tumor cells exclusively and fluoresce once resident therein. Thus the fluorescence is analyzed quantitatively, such that, since the fluorescent drug resides exclusively in the tumor cells, the quantitative measurement of the drug concentration is a measure of the concentration of tumor cells. This allows the MR imaging is used to provides a more complete picture of the amount of tumor cells present.

The control system can be operated so that the image representing the residual tumor mass is segmented and the data transferred to the robot which is used to resect the tumor to the level assigned by the quantitative analysis of the fluorescent images.

On the display, the MR images which are co-registered with the fluorescent images are involved in the segmentation to provide tumor cell zones and also keep out zones related to eloquent and sensitive brain structures.

The control 56 is operated so that the imaging rate for the fluorescence imaging is of the order of 30 frames per second so it allows the resection to be monitored as it occurs in real time.

The control system 10 of the robot is programmed to stop movement of the robot as each MRI image is recorded.

The MR control 20 is arranged such that the MR imaging includes diffusion tensor imaging which shows on the image all the fiber tracks in the brain and of particularly importance, those around the tumor.

The fluorescent chemicals injected which attach to the tumor cells can also contain MRI markers so that they appear on both the MR images and the fluorescence images.

The registration system is arranged such that the registration of the images is achieved by placing markers on a tool of the robot or on surgical instruments.

Since various modifications can be made in my invention as herein above described, and many apparently widely different embodiments of same made within the spirit and scope of the claims without department from such spirit and scope, it is intended that all matter contained in the accompanying specification shall be interpreted as illustrative only and not in a limiting sense.

## Claims

1. Apparatus for viewing a part of a patient comprising:
an optical assembly for receiving light from the part of the patient, the optical assembly including stereoscopic viewing components arranged for use in generating 2D and 3D images, the optical assembly being adjustable to change at least a field of view;
a display for viewing of images generated from the light received from the part;
and a control system for controlling the optical assembly and for generating the images;
a communication arrangement for communicating between the optical assembly and the processing system;
wherein the optical assembly includes a mount arranged to locate the assembly within a bore of an MRI magnet;
and wherein the optical assembly, control system and the communication arrangement are compatible with the MRI magnet so as to allow simultaneous communication and MR imaging.

2. The apparatus according to claim 1 wherein the control system is arranged to change the viewing parameters of the optical assembly including one or more of zoom, depth of field, focus, pan, tilt, window levelling, color, balance, magnification.

3. The apparatus according to any preceding claim wherein an illumination source is integrated into the optical assembly to illuminate viewing of the part.

4. The apparatus according to claim any preceding claim wherein there is provided an imaging/encoding device for encoding light from the optical assembly from the part into digital information and wherein the imaging/encoding device is located near the patient in the bore of a magnet with the optical assembly and the control system is located outside of the bore.

5. The apparatus according to claim any preceding claim wherein there is provided an imaging/encoding device for encoding light from the optical assembly from the part into digital information and wherein the imaging/encoding device is located remotely from the optical assembly and the communication arrangement comprises a fiber optic system.

6. The apparatus according to any preceding claim wherein there is provided an imaging/encoding device for encoding light from the optical assembly from the part into digital information and wherein the imaging/encoding device is located remotely from the optical assembly and the communication arrangement comprises a light tube movable with the optical assembly.

7. The apparatus according to any preceding claim wherein the optical assembly, control system and the communication arrangement are made compatible with the MRI magnet by one or more of:
an RF filter on electrical communication cables to prevent stray RF from the electrical communication cables signals from effecting the imaging;
an RF filter on electrical communication cables to prevent the RF imaging signals from affecting the imaging/encoding device;
an RF enclosure around the optical assembly and the imaging/encoding device;
the optical assembly and imaging/encoding device being formed of materials which are compatible with the magnetic field;
cable traps on electrical communication cables to prevent heating thereof in the RF field of the imaging system;
a magnetic shield around or adjacent the components to prevent the magnetic field from affecting the components.

8. The apparatus according to claim any preceding claim wherein the apparatus is used with a surgical robot system including at least one robotic arm with at least one end effector for operating one or more surgical tools and wherein the optical assembly is mounted on the robotic arm or on the tool so as to be moveable therewith.

9. The apparatus according to claim 8 wherein the optical assembly is mounted on a support arm separate from the robotic arm or arms so as to be moveable with the support arm and wherein the support arm is movable so as to avoid interference with the robotic arm or arms.

10. The apparatus according to claim 8 or 9 wherein the control system is arranged to operate the optical assembly to change one or more of the viewing parameters thereof in response to movement of the robotic arm.

11. The apparatus according to claim 8, 9 or 10 wherein the control system is arranged to operate the optical assembly to change one or more viewing parameters of focal depth and focal position thereof in response to movement of the tool relative to the optical assembly.

12. The apparatus according to any one of claims 8 to 11 wherein the robotic arm or arms are controlled to be moved in response to input from a user and wherein the movement of the arm or arms is scaled in relation to the image displayed.

13. The apparatus according to any one of claims 8 to 12 wherein the robotic arm or arms are controlled to be moved in response to input from a user and wherein the movement of the arm or arms is limited by no-touch zones indicated on the image.

14. The apparatus according to any one of claims 8 to 13 wherein the MRI system is arranged to generate MR images and wherein the control system is arranged to cooperate with a control system of the MRI in order to overlay the MR images on the visual images on the display and wherein the control system of the MRI is arranged to operate in response to changes in the visual image displayed and/or movement of the tool.

15. The apparatus according to any one of claims 8 to 14 wherein the control system of the MRI is arranged to operate to trigger a scan and/or to change one or more of:
the scan parameters of the MR images including one or more of resolution, slice thickness and dimension;
the scan type;
the part of the patient / anatomy being scanned;
detecting a position of the tool tip to providing an anatomical image.

16. The apparatus according any one of claims 8 to 15 wherein the control system is integrated with an IGS system which can overlay or augment the surgeon's view with image guidance data.

17. The apparatus according to any one of claims 8 to 16 wherein the robot is programmed to stop as each MRI image is recorded.

18. The apparatus according to any one of claims 8 to 17 wherein the MR imaging is carried out including diffusion tensor imaging which shows on the image all the fiber tracks in the brain and of particularly importance, those around the tumor.

19. The apparatus according to any one of claims 8 to 18 wherein the registration of the images is achieved by placing markers on a tool of the robot or on a surgical instrument.
